# EUROPEAN PATENT SPECIFICATION

(11) **EP 1 925 310 B1**
(45) Date of publication and mention of the grant of the patent: **25.03.2015**
(21) Application number: 06024463.9
(22) Date of filing: 24.11.2006
(51) Int. Cl.: A61K 36/23, A61K 36/53, A61P 17/02

(54) **Plant extracts from Plectranthus and Centella for treating skin disorders and accelerating wound healing in diabetic patients**
Pflanzenextrakte aus Plectranthus und Centella zur Behandlung von Hautkrankeiten und zur Beschleunigung der Wundheilung bei Diabetikern
Extraits de Plectranthus et Centella pour les traitment des disordres de la peau et accelerer la guérison des lésions en patients diabétiques

(43) Date of publication of application: 28.05.2008
(73) Proprietor: DEVELOPMENT CENTER FOR BIOTECHNOLOGY, Xizhi City, Taipei County (TW)
(72) Inventor: Wu, Rey-Yuh, Xizhi City Taipei County (TW); Chung, Yuh-Shan, Xizhi City Taipei County (TW); Wu, Yu-Yuan, Xizhi City Taipei County (TW); Siu, Ma-Li, Xizhi City Taipei County (TW); Huang, Hung-Jen, Xizhi City Taipei County (TW); Hsiao, Chin-Wen, Xizhi City Taipei County (TW)
(74) Representative: Becker Kurig Straus

(56) References cited:
- US-A- 5 834 437
- US-A1- 2006 099 283
- SHUKLA A ET AL: "In vitro and in vivo wound healing activity of asiaticoside isolated from Centella asiatica" JOURNAL OF ETHNOPHARMACOLOGY, ELSEVIER SCIENTIFIC PUBLISHERS LTD, IE, vol. 65, no. 1, April 1999 (1999-04), pages 1-11, XP002240115 ISSN: 0378-8741
- LUKHOBA ET AL: "Plectranthus: A review of ethnobotanical uses" JOURNAL OF ETHNOPHARMACOLOGY, ELSEVIER SCIENTIFIC PUBLISHERS LTD, IE, vol. 103, no. 1, 3 January 2006 (2006-01-03), pages 1-24, XP005175868 ISSN: 0378-8741
- DATABASE EMBASE [Online] ELSEVIER SCIENCE PUBLISHERS, AMSTERDAM, NL; 2001, INCANDELA L ET AL: "Total triterpenic fraction of Centella asiatica in chronic venous insufficiency and in high-perfusion microangiopathy" XP002427324 Database accession no. EMB-2001365167 & ANGIOLOGY 2001 UNITED STATES, vol. 52, no. 10 SUPPL. 2, 2001, pages S9-S13, ISSN: 0003-3197
- DATABASE EMBASE [Online] ELSEVIER SCIENCE PUBLISHERS, AMSTERDAM, NL; 2001, CESARONE M R ET AL: "Evaluation of treatment of diabetic microangiopathy with total triterpenic fraction of Centella asiatica: A clinical prospective randomized trial with a microcirculatory model" XP002427325 Database accession no. EMB-2001365174 & ANGIOLOGY 2001 UNITED STATES, vol. 52, no. 10 SUPPL. 2, 2001, pages S49-S54, ISSN: 0003-3197
- DATABASE EMBASE [Online] ELSEVIER SCIENCE PUBLISHERS, AMSTERDAM, NL; 2001, INCANDELA L ET AL: "Treatment of diabetic microangiopathy and edema with total triterpenic fraction of Centella asiatica: A prospective, placebo-controlled randomized study" XP002427326 Database accession no. EMB-2001365170 & ANGIOLOGY 2001 UNITED STATES, vol. 52, no. 10 SUPPL. 2, 2001, pages S27-S31, ISSN: 0003-3197

## Description

### FIELD OF THE INVENTION

The present invention relates to Chinese herbal medicine extracts. Specifically, the present invention relates to the use of the extracts of *Plectranthus amboinicus* Benth and *Centella asiatica* Urban for treating skin disorders, including enhancing the healing of wounds for diabetic patients.

### BACKGROUND OF THE INVENTION

Plectranthus *amboinicus* Benth (Patchouli), growing in Malaysia and India, is a decorative medicinal herb commonly cultivated by common families. The medicinal part of *Plectranthus amboinicus* Benth is the epigeal portion. *Plectranthus amboinicus* Benth is also known as Cuban oregano, Indian borage, Indian mint, Mexican mint, Mexican oregano or Spanish thyme. The Eastern Indians use *Plectranthus amboinicus* Benth as fabric aromatics, and the English discovered its attractive fragrance when importing shawl fabrics from India in the 1820's. When the *Plectranthus amboinicus* Benth leaves are put directly with clothing, it not only has an aromatic effect, but also prevents the clothing from being eaten by moths. It is thought to have uses for disinfection, excitement and preventing insect bites. In addition, *Plectranthus amboinicus* Benth may be used for treating poisonous snake bites, and relieving symptoms such as headache, flatulence, vomiting, diarrhea and fever. Moreover, *Plectranthus amboinicus* Benth oil is a popular perfume in Asia, and it is used for improving epithelia regeneration, treating acne, and relieving the symptoms of eczema, Athlete's foot and dry cracking skin in aromatherapy. Furthermore, *Plectranthus amboinicus* Benth is a good tranquilizer and aphrodisiac that can relieve anxiety and enhance sexual desire.

*Centella asiatica* Urban (pennywort) is a plant naturally growing in the coast area of Madagascar and the Indian Ocean. The medicinal part of *Centella asiatica* Urban of the Apiaceae family is its dried whole plant. *Centella asiatica* Urban is also known as European water-marvel, Gotu kola, Kola, Indian pennywort, Indian ginseng, Horse-hoof grass, Pegaga, Mandookaparni, Tiger herbal, Spadeleaf, or Tono. For hundreds of years, *Centella asiatica* Urban has been thought to be useful in the traditional medicines for improving wound healing in Asian regions. The extracts of *Centella asiatica* Urban comprise two major compounds: asiaticoside and madecassic acid. The uses of *Centella asiatica* Urban extracts are in treating burn wounds, trauma, and preventing postoperative adhesion, and the preparation methods of *Centella asiatica* Urban extracts have been described in some patent applications. For example, US 4,318,906 and CN 1353972A disclose the medical uses of *Centella asiatica* Urban as the single active ingredient; US 6,475,536, US 6,267,996, CN 1313124A, CN 93110425.4 and CN 1089497A disclose the use of *Centella asiatica* Urban in combination with other substances for cosmetic formulation, treating bums or making ointment for trauma; and US 5,834,437, US 6,417,349 and CN 1194154A disclose the methods for preparing *Centella asiatica* Urban extracts. In US 5,834,437 asiatic acid derivatives are disclosed to have wound-healing properties. US 2006/099283 relates to a composition comprising the juice of a leaf of *Plecanthrus amboinicus* in the treatment of a tumor. Shukla et al. ("In vitro and in vivo wound healing activity of asiaticoside isolated from Centella asiatica", Journal of Ethnopharmacology, Elsevier Scientific Publishers, Ltd. IE; Vol. 65 No. 1, April 1999, p. 1-11) discloses the activity of a compound, asiaticoside, isolated from *Centella asiatica* in the promotion of wound healing.

The functions of skin include protection, excretion, secretion, absorption, heat regulation, storage, sensation and immune process regulation. When a wound occurs on the skin, for example, a wound caused by general trauma or a bedsore, it will cause an adverse effect on the skin and interfere with its normal functions. Diabetes mellitus is a common and chronic worldwide disease. According to the statistical report of the Department of Health, Executive Yuan, Taiwan, R.O.C. published in 2002, mortality due to diabetes increased from 7.9 deaths per 100,000 persons in 1980 to 39.3 deaths per 100,000 persons in 2002. The most common diabetes complications are neuropathy and angiopathy. Autonomic neuropathy causes reduction of sweating and results in dry cracking skin. Sensory neuropathy makes the patient insensitive to pain. Angiopathy causes macro- or micro-vascular obstructions, poor circulation, and atrophy of the skin. Once the wound is infected in a diabetic patient, it is difficult to be treated because not only controlling the bacterial infection but also the healing of the wound must be achieved. The only commercial therapeutic medicine available on the market for healing of wounds for diabetic patients is the gene recombinant product PDGF Regranex Gel (Becaplermin 0.01%) and it is very expensive. Therefore, it is necessary to provide new medicaments for enhancing the healing of wounds for diabetic.

### SUMMARY OF THE INVENTION

One of the purposes of the present invention is to provide a pharmaceutical composition for treating skin disorders (including enhancing the healing of wounds for diabetic patients), comprising a therapeutically effective amount of *Plectranthus amboinicus* Benth crude extracts/extracts and a therapeutically effective amount of *Centella asiatica* Urban whole plant extracts comprising asiaticoside.

Another purpose of the present invention is to provide a use of a combination of *Plectranthus* amboinicus Benth crude extracts/extracts and *Centella asiatica* Urban whole plant extracts comprising asiaticoside for the manufacture of a medicament for treating skin disorders.

Still another purpose of the present invention is to provide a method for preparing the pharmaceutical composition of this invention.

A further purpose of the present invention is to provide a wound dressing comprising the pharmaceutical composition of the invention.

The present invention is described in detail in the following sections. Other characterizations, purposes and advantages of the present invention can be easily found in the detailed descriptions and claims of the invention.

### BRIEF DESCRIPTION OF THE DRAWINGS

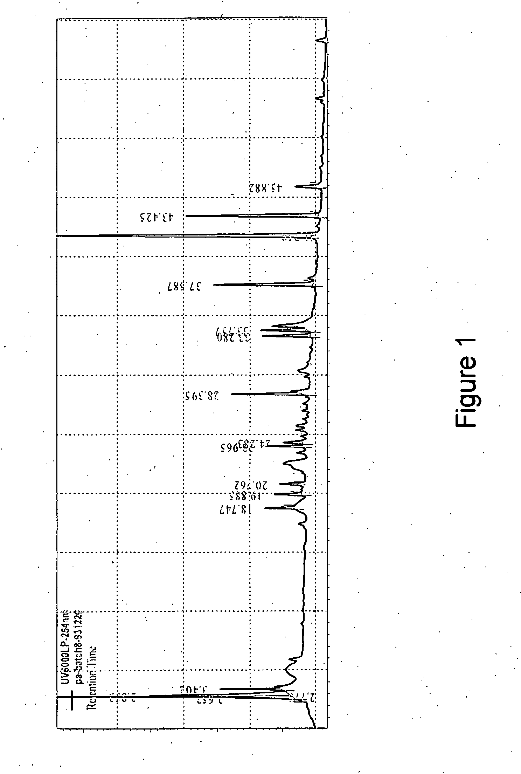

**Figure 1** shows the chromatography spectrum of extracts (PAet) extracted from alcohol-dipped, dried *Plectranthus amboinicus* Benth by Reverse Phase HPLC at UV 254nm.

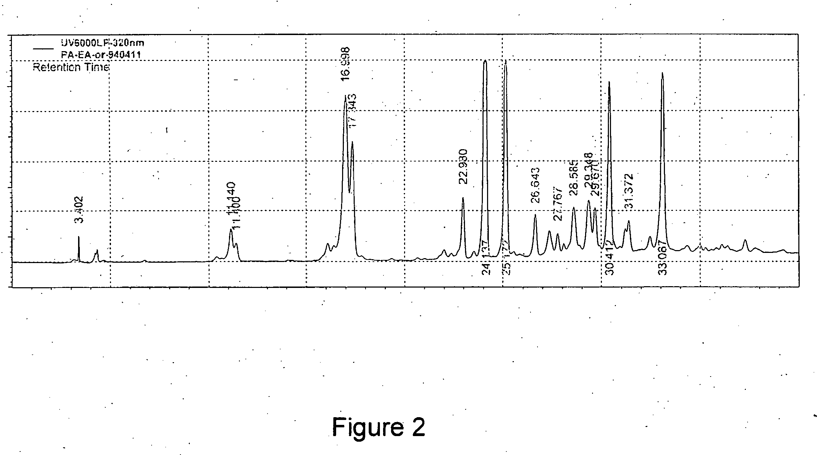

**Figure 2** shows the chromatography spectrum of column purified PA extracts (PA-F4) from alcohol-dipped, dried *Plectranthus amboinicus* Benth by Normal Phase HPLC at UV 320 nm.

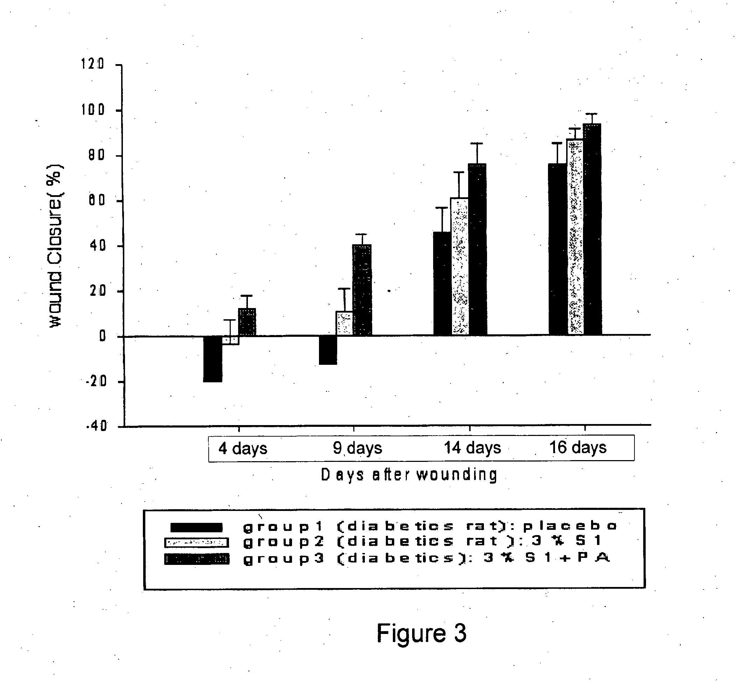

**Figure 3** shows wound closures in STZ-induced diabetic rats treated with *Centella asiatica* Urban extracts (S1) and *Plectranthus amboinicus* Benth (PA) crude extracts. Group 1: wound applied with ointment without medicament; group 2: wound applied with ointment containing 3% S1; group 3: wound applied with ointment containing 3% S1 + 2% PA crude extracts.

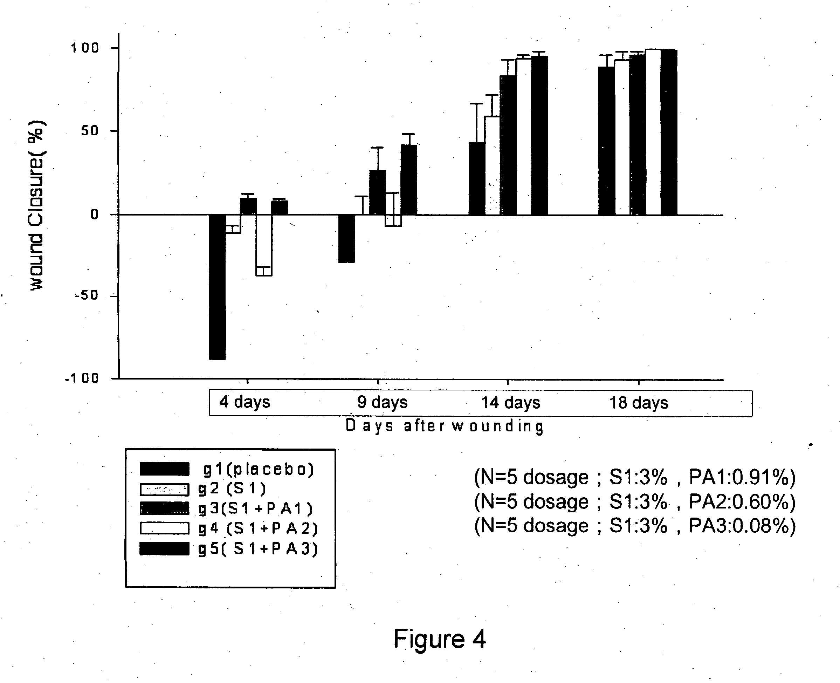

**Figure 4** shows wound closures in STZ-induced diabetic rats treated with S1 and PA extracts (PA1, PA2 and PA3) extracted with different solvents. Group 1: wound applied with ointment without medicament; group 2: wound applied with ointment containing 3% S1; group 3: wound applied with ointment containing 3% S1 + PA1 extracts; group 4: wound applied with ointment containing 3% S1 + PA2 extracts; group 5: wound applied with ointment containing 3% S 1 + PA3 extracts.

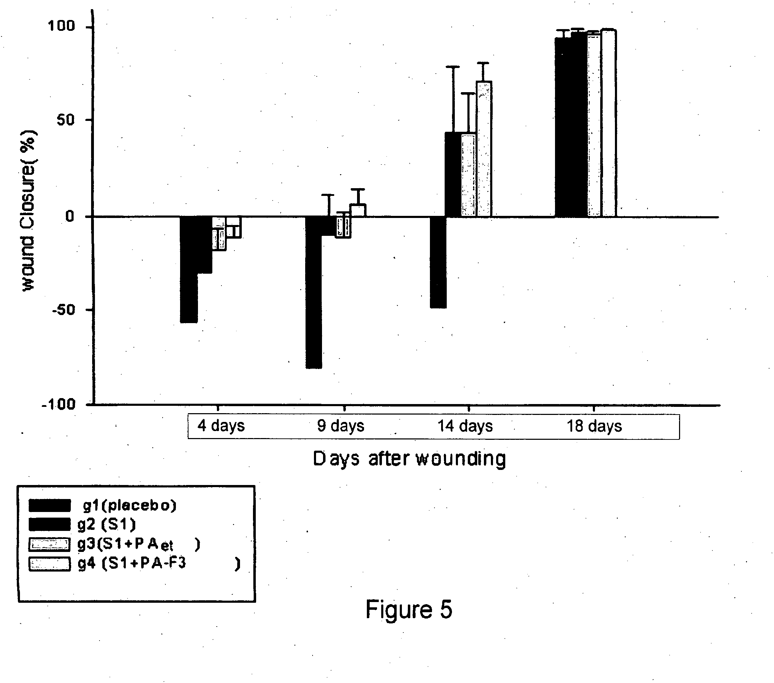

**Figure 5** shows wound closures in STZ-induced diabetic rats treated with S1, alcohol extracted PA extracts (PAet) and partially column purified PA extracts (PA-F3). Group 1: wound applied with ointment without medicament; group 2: wound applied with ointment containing 3% S1; group 3: wound applied with ointment containing 3% S1 + PAet extracts; group 4: wound applied with ointment containing 3% S 1 + PA-F3 extracts.

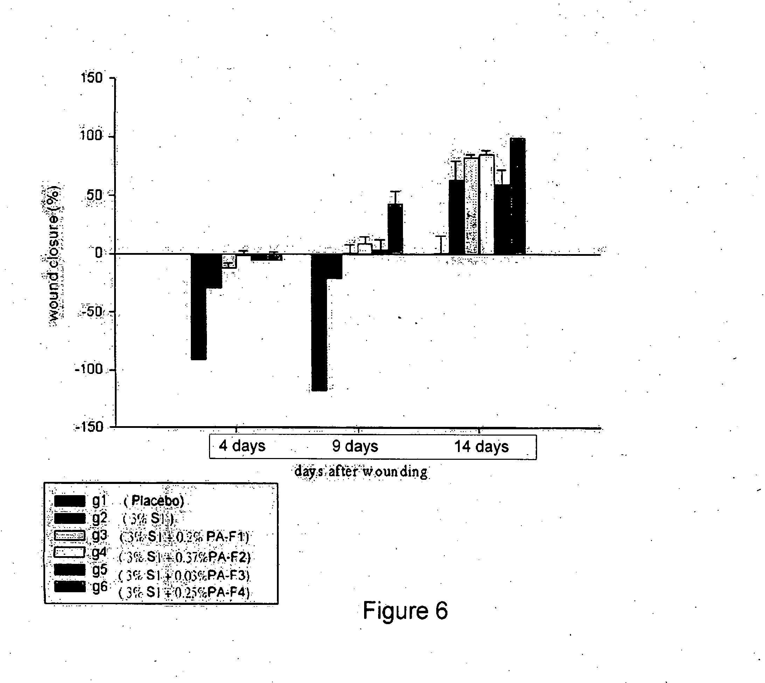

**Figure 6** shows wound closures in STZ-induced diabetic rats treated with S1 and column purified PA extracts (PAet, PA-F1, PA-F2, PA-F3 and PA-F4). Group 1: wound applied with ointment without medicament; group 2: wound applied with ointment containing 3% S1; group 3: wound applied with ointment containing 3% S1 + 0.2% PA-F1 extracts; group 4: wound applied with ointment containing 3% S1 + 0.37% PA-F2 extracts; group 5: wound applied with ointment containing 3% S1 + 0.03% PA-F3 extracts; group 6: wound applied with ointment containing 3% S1 + 0.25% PA-F4 extracts.

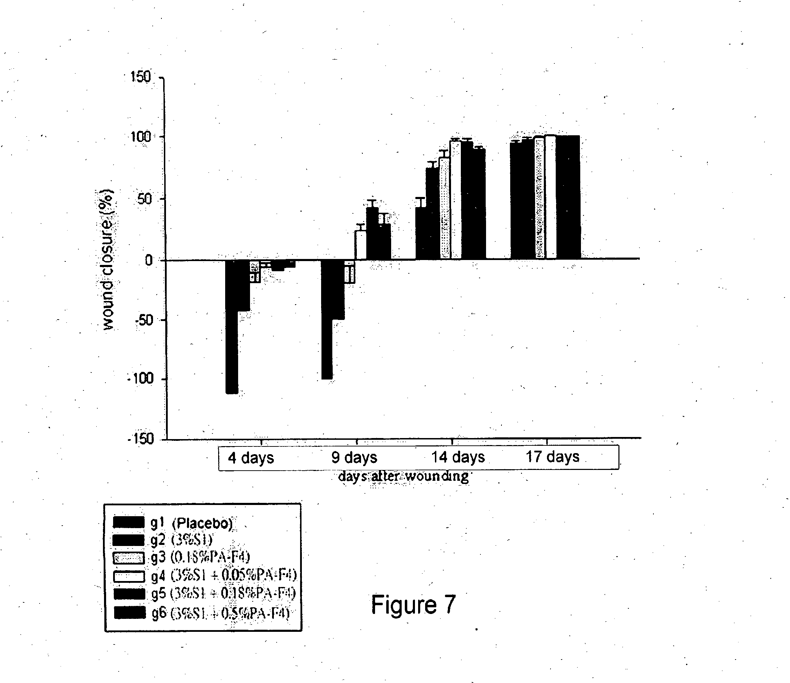

**Figure 7** shows wound closures in STZ-induced diabetic rats treated with S1 and PA-F4 extracts of different concentration. Group 1: wound applied with ointment without medicament; group 2: wound applied with ointment containing 3% S1; group 3: wound applied with ointment containing 0.18% PA-F4 extracts; group 4: wound applied with ointment containing 3% S1 + 0.05% PA-F4 extracts; group 5: wound applied with ointment containing 3% S1 + 0.18% PA-F4 extracts; group 6: wound applied with ointment containing 3% S1 + 0.5% PA-F4 extracts.

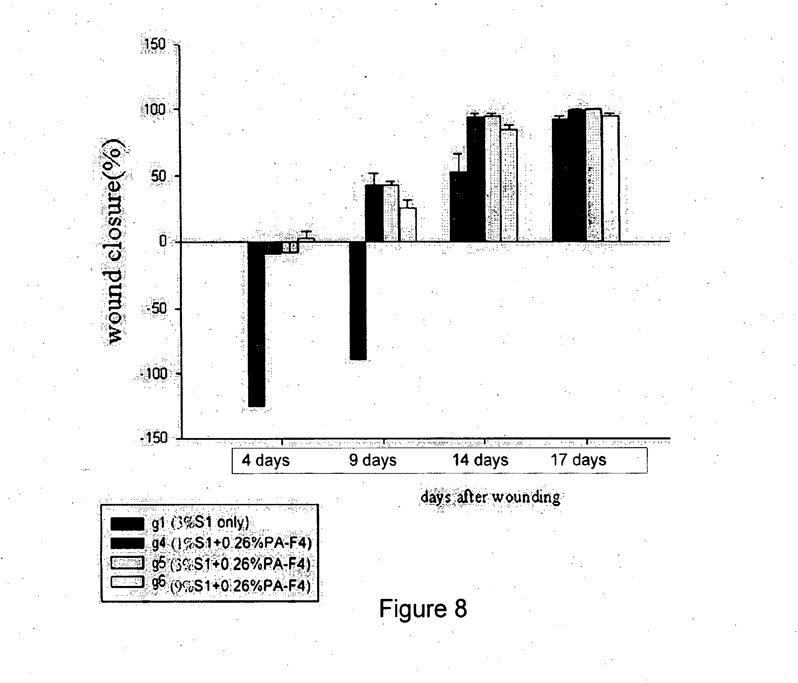

**Figure 8** shows wound closures in STZ-induced diabetic rats treated with PA-F4 extracts and S1 of different concentration. Group 1: wound applied with ointment containing 3% S1; group 4: wound applied with ointment containing 1% S1 + 0.26% PA-F4 extracts; group 5: wound applied with ointment containing 3% S1 + 0.26% PA-F4 extracts; group 6: wound applied with ointment containing 9% S1 + 0.26% PA-F4 extracts.

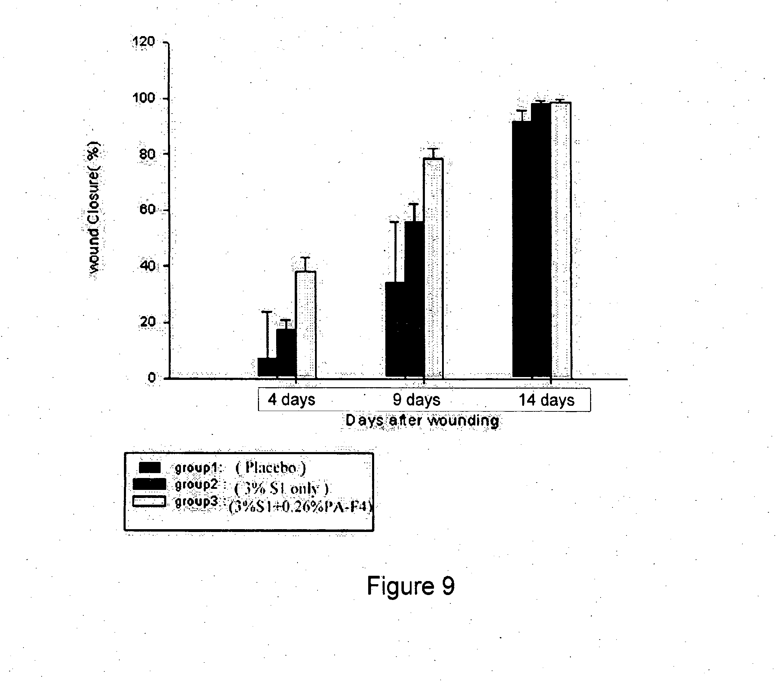

**Figure 9** shows wound closures in diabetic db/db mice treated with S1 and PA-F4 extracts. Group 1: wound applied with ointment without medicament; group 2: wound applied with ointment containing 3% S1; group 3: wound applied with ointment containing 3% S1 + 0.26% PA-F4 extracts.

### DETAILED DESCRIPTION OF THE INVENTION

The term "skin disorders" as used herein includes wounds or sores. In one embodiment, skin disorders include a cut, laceration, abrasion, stab or other similar skin injuries, preferably a diabetic patient's wound; sores include bedsores.

The term "treating" or "enhancing" as used herein denotes improving the symptoms.

The term "patients" as used herein denotes animals, especially mammals. In one preferred embodiment, the term "patients" denotes "humans."

The term "therapeutically effective amount" as used herein refers to the amount of the pharmaceutical composition used alone or in combination with other medicaments for treating disorders that shows therapeutic efficacy.

The term "carrier" or "pharmaceutically acceptable carrier" refers to diluents, excipients, acceptors or analogues, which are well known to persons of ordinary skill in the art for manufacturing pharmaceutical compositions.

The term *"Centella asiatica* Urban extracts" denotes extracts of dried Centella *asiatica* Urban whole plants, wherein the major active components comprise asiaticoside and madecassic acid. Preferably, it is the medicinal part available on the market that mainly comprises asiaticoside and madecassic acid, and its purity is greater than 70%.

The term "Plectranthus amboinicus Benth crude extracts" or *"Plectranthus amboinicus* Benth extracts" denotes extracts obtained from extracting the epigeal portion of *Plectranthus amboinicus* Benth.

The present invention is characterized by using the combination of *Plectranthus amboinicus* Benth crude extracts/extracts and *Centella asiatica* Urban whole plant extracts comprising asiaticoside for treating skin disorders including enhancing the healing of wounds for diabetic patients.

The present invention herein provides a pharmaceutical composition for treating skin disorder (including enhancing the healing of wounds for diabetic patients), comprising a therapeutically effective amount of *Plectranthus amboinicus* Benth crude extracts/extracts and a therapeutically effective amount of *Centella asiatica* Urban whole plant extracts comprising asiaticoside. Said pharmaceutical composition can be used for treating disorders including but not limited to wounds and sores. In one preferred embodiment, said disorders are general trauma and bedsores, more preferably a diabetic patient's wound.

The pharmaceutical composition of the present invention can be applied topically to the wounds, and it may be formulated as a spray or non-spray. A spray form includes spray or solution; a non-spray form may be semi-solid or solid, preferably a solid form having a kinematic viscosity greater than water. Suitable formulations include but are not limited to suspensions, emulsions, creams, ointments, liniments and the like. If necessary, it may be sterilized or mixed with any pharmaceutically acceptable carriers, for example, stabilizers, wetting agents and the like. Preferably, the pharmaceutical composition of the invention is formulated as ointments, wherein the preferred pharmaceutically acceptable carriers include but are not limited to higher fatty acids, waxes, lipids, glycerol, higher alcohols or synthetic lipids. The pharmaceutical composition of the present invention, no matter which form it is formulated in, may further comprise emollients, fragrances or colors to increase the acceptability for various uses.

The pharmaceutical composition of the present invention may be conveniently used for manufacturing a wound dressing comprising a therapeutically effective amount of *Plectranthus amboinicus* Benth crude extracts/extracts and a therapeutically effective amount of *Centella asiatica* Urban whole plant extracts comprising asiaticoside, wherein said wound dressing includes but is not limited to a bandage with adhesive, plaster patch and the like.

The pharmaceutical composition of the present invention may be prepared by persons of ordinary skill in the art using conventional methods. One embodiment of the present invention includes a method of preparing an ointment comprising the following steps:
- heating the ointment base (BETAMETHASONE ointment base, Sinphar; Taiwan) in a water bath at about 50°C until softened;
- adding the softened ointment base in turn into the beaker containing frozen dried *Plectranthus amboinicus* Benth extracts;
- adjusting the amount of ointment base and *Plectranthus amboinicus* Benth extract, the weight ratio being 99.75:0.25, to make the 0.25% *Plectranthus amboinicus* Benth extract ointment;
- homogenously mixing 0.25% *Plectranthus amboinicus* Benth extract ointment and *Centella asiatica* Urban whole plant extracts comprising asiaticoside in a weight ratio of 97:3 to make the 3% *Centella asiatica* Urban extract + 0.25% *Plectranthus amboinicus* Benth extract ointment; and
- storing the ointment at 4°C in a refrigerator.

The preparation method for the *Centella asiatica* Urban whole plant extracts comprising asiaticoside, the active component in the pharmaceutical composition of the present invention, is well known to persons of ordinary skill in the art of herbal medicine extraction. A suitable method comprises:
- extracting *Centella asiatica* by ethanol reflux extraction for 2 hours, and repeating it 2 to 3 times;
- condensing the ethanol extract fluids to obtain the extracts;
- retrieving the ethanol extracts, mixing and dissolving the extracts in water;
- extracting with petroleum ether, chloroform, ethyl acetate separately, and then extracting with water-saturated n-butanol;
- retrieving the solvent of n-butanol extracts under reduced pressure to obtain a cream-like substance and dissolving the substance in a small amount of methanol;
- adding anhydrous acetone, several-fold, to the solution, to precipitate a yellowish crude total glucoside, drying and weighting the sediments;
- a small amount of methanol is added to dissolve the crude total glucoside, mixing properly with silica gel and drying the mixture;
- wet column packing with silica gel for chromatography and loading the sample silica gel;
- eluting with chloroform, methanol and water (16:6:1 homogenous mixture) and collecting 18 components in turn; each component is 50 ml and the components are thin-layer spotting to compare with known controls;
- combining components 9 to 15, concentrating, and then purifying with column using chloroform, methanol and water (14:6:1 homogenous mixture) for elution; and
- concentrating the same components and crystallizing with methanol repeatedly to obtain the white aciculate crystalline asiaticoside.

Furthermore, the drug substance contains asiaticoside and madecassic acid, with a purity greater than 70%, is available on the market.

Disclosed herein are also the preparation methods for *Plectranthus amboinicus* Benth crude extracts and extracts.

### Preparation of Plectranthus amboinicus Benth crude extracts

Wash the fresh drug substances of *Plectranthus amboinicus* Benth with water, then extract the juice with a juice extractor. Freeze-dry the *Plectranthus amboinicus* Benth juice to obtain dry powders. Use a suitable solvent, for example, chloroform or methanol, to extract the *Plectranthus amboinicus* Benth crude extracts.

### Preparation of Plectranthus amboinicus Benth extracts

Dip a fixed amount of *Plectranthus amboinicus* Benth dry materials in a proper amount of high-polarity solvent. After filtration, dip again with a proper amount of high-polarity solvent and concentrate the *Plectranthus amboinicus* Benth extract solution with a reduced pressure rotary evaporator until its volume is 2-3% of the original volume. Dilute with solvent and separate by column. Optionally, it can be eluted continuously with four different solvents comprising high- to low-polarity solvents (namely high-polarity solvent, sub-high-polarity solvent, medium polarity solvent and low-polarity solvent). According to the present invention, high-polarity solvents include but are not limited to water, methanol, isopropanol, or a mixture of two or more of the preceding solvents. Low-polarity solvents include but not are limited to chloroform, acetone, ethyl acetate, or a mixture of two or more of the preceding solvents. Preferably, column chromatography separation is conducted using methanol-treated DIAION column. For example, dip DIAION, which has the same weight as the dried *Plectranthus amboinicus* Benth, in methanol, and pack the methanol-treated DIAION into chromatography column. After pack, washing DIAION with methanol (one- to two-fold volume) then wash DIAION with double distilled water (five- to six-fold volume) and the packing is done.

### Composition analysis of the Plectranthus amboinicus Benth extracts

Instruments and equipment
High Performance Liquid Chromatography (HPLC)
Pump: Spectra-Physics P4000
Detector: UV/VIS Spectra-PhysicsSpectraSystem UV600OLP
Auto sampler: Thermo Separation Pruducts AS3500
Software: Thermo Separation Pruducts Chrom Quest
System Controller: Thermo Separation Pruducts SN4000

### 1. Condition for Normal Phase HPLC

- Chromatography column:: Phenomenex, 4.6x250nm, Luna 5u silica(2)
- Flow rate: 1.0 ml/min: Pressure Limit: 250 kgf/cm²
- Sample amount:: 10 µl
- PDA conditions:: Sampling period: 0.64 sec
Wavelength range: 190-370 nm
Channels: 270, 320 nm

### Elution Profile:

| | Time (min) | | | |
|---|---|---|---|---|
| Mobile phase | 0 | 15 | 45 | 50 |
| n-hexane | 95% | 85% | 30% | 95% |
| Ethyl acetate | 5% | 15% | 70% | 5% |

### 2. Condition for Reverse Phase HPLC

- Chromatography column:: COSMOSIL, 4.6x250nm, SC15-MS
- Flow rate:: 1.0 ml/min ²
- Pressure Limit:: 250 kgf/cm
- Sample amount:: 10 µl
- PDA conditions:: Sampling period: 0.64 sec
Wavelength range: 190-370 nm
Channels: 204, 254 nm

### Elution Profile:

| | Time (min) | | | |
|---|---|---|---|---|
| Mobile phase | 0 | 10 | 55 | 60 |
| H₂O | 90% | 90% | 20% | 80% |
| Acetonitrile | 10% | 10% | 80% | 20% |

According to the present invention, an ointment containing *Plectranthus amboinicus* Benth extracts and *Centella asiatica* Urban whole plant extracts comprising asiaticoside shows higher efficacy in enhancing healing of wounds than ointment containing *Plectranthus amboinicus* Benth crude extract and *Centella asiatica* Urban whole plant extracts comprising asiaticoside (shown in the example below). The optimum dosages of *Plectranthus amboinicus* Benth extracts and *Centella asiatica* Urban whole plant extracts comprising asiaticoside in the ointment are about at least 0.01% and about at least 0.1% by weight respectively; preferably, *Plectranthus amboinicus* Benth extracts are about 0.01% to 5% by weight of the ointment and the *Centella asiatica* Urban whole plant extracts comprising asiaticoside are about 0.1% to 20% by weight of the ointment, and the weight ratio between *Plectranthus amboinicus* Benth extracts and *Centella asiatica* extracts is about 1:60 to 1:4.

Persons skilled in the art should easily choose the suitable routes and the dosages for treatments. A preferred route is topical administration. Dosage will depend on the nature and condition of the disorders, ages and health conditions of the patients, administration routes and any previous therapy. Persons skilled in the art should know that the dosage may vary depending on the individual's age, size, health condition and other related factors.

The invention is described in detail in the following examples. The following procedures are used to prove the effects of the composition comprising *Plectranthus* amboinicus Benth crude extracts or extracts and *Centella asiatica* Urban extracts in treating skin disorders, especially enhancing the healing of wounds for diabetic patients.

### Example 1. Plectranthus amboinicus Benth crude extracts obtained from direct extraction

1.25g fresh *Plectranthus amboinicus* Benth was washed with water and juices were extracted with a juice extractor. The volume of the *Plectranthus amboinicus* Benth juices, measured by a graduated cylinder, is 1050 ml. The *Plectranthus amboinicus* Benth juices were freeze-dried and 19g dry powders were obtained; the yield is 1.5%.

### Example 2. Extracting Plectranthus amboinicus Benth extracts (named hereinafter PA1, PA2 and PA3) using different solvents

0.5kg fresh *Plectranthus amboinicus* Benth was washed with water and juices were extracted with a juice extractor. 200ml *Plectranthus amboinicus* Benth juices, measured by a graduated cylinder, were freeze-dried and 4.4g dry powders were obtained; the yield is 0.9%.

4.4g *Plectranthus amboinicus* Benth dry powders were added into 44ml low-polarity solvents and extracted for 24 hours; the process was repeated twice. The two fluids extracted using the low polarity solvents were concentrated and dried, and 0.15g PA3 was obtained; the yield is 3.4%.

The remaining *Plectranthus amboinicus* Benth dry powders, which were not extracted by the preceding step, were extracted again with 44ml sub-high-polarity solvents for 24 hours; the process was repeated twice. The two fluids extracted using the sub-high-polarity solvents were concentrated and dried, and 1.19g PA2 was obtained; the yield is 27%.

The remaining *Plectranthus amboinicus* Benth dry powders, which is not extracted by the proceeding step, were extracted again with 44ml high polarity solvents for 24 hours, repeated twice. The two fluids extracted using the sub-high polarity solvents were concentrated and dried, and 1.83g PA1 was obtained; the yield is 41.6%.

### Example 3. Plectranthus amboinicus Benth extracts (PAet, PA-F1, PA-F2, PA-F3 and PA-F4) purified by chromatography column separation

2kg *Plectranthus amboinicus* Benth dry materials were dipped in ten-fold high-concentration alcohol for 24 hours. After filtration and a secondary dipping with ten-fold high-concentration alcohol for 24 hours, the *Plectranthus amboinicus* Benth extract fluids were concentrated by a reduced pressure rotary evaporator until the volume was 2-3% of the original volume (if dried, the powder is PAet, the weight is 30g, the yield is 1.5%, and the chromatography spectrum is shown in Figure 1).

The product obtained from the preceding step was diluted with solvent and loaded into a treated DIAION column. The column was eluted with double distilled water (ten-fold dry material volume) and a sample (labeled as PA-F1) was collected; the weight is 8.0g and the yield is 0.4%.

Then, the column was eluted with medium-concentration alcohol (five- to ten-fold dry material volume) and a sample (labeled as PA-F2) was collected; the weight is 10.4g and the yield is 0.52%.

The column was eluted again with high-concentration alcohol (five- to ten-fold dry material volume) and a sample (labeled as PA-F3) was collected; the weight is 16g and the yield is 0.8%.

Finally, the column was eluted with a solvent mixture of alcohol and ethyl acetate (five- to ten-fold dry material volume) and a sample (labeled as PA-F4) was collected; the weight is 13g and the yield is 0.65%. The chromatography spectrum is shown in Figure 2.

### Example 4. Preparation for the ointments containing Centella asiatica Urban extracts and/or Plectranthus amboinicus Benth extracts using ointment base

1g *Centella asiatica* Urban extract powders were added into 99g ointment base, which was preheated in a water bath at about 50°C until softened, to make the 1% *Centella* asiatica extract ointment.

The weight ratio of ointment base to *Plectranthus amboinicus* Benth extract was 99.75:0.25. The ointment base was preheated in a water bath at about 50°C until softened and added in turn into the beaker containing frozen dried *Plectranthus amboinicus* Benth extracts to make the 0.25% *Plectranthus amboinicus* Benth extract ointment. 0.25% *Plectranthus amboinicus* Benth extract ointment was mixed with *Centella asiatica* Urban extracts in a weight ratio of 99:1 to make the 1% *Centella asiatica* extract + 0.25% *Plectranthus amboinicus* Benth extract ointment.

### Example 5. Animal experiments for wound closures in diabetic rats

### [Animal experiments]

### Induction of high blood sugar in the animals

Rats were purchased and the high-blood-sugar induction with Streptozotocin (STZ) was conducted after their weights were over 300g. The animals successfully induced to have high blood sugar (over 300mg/DL) were selected to conduct the wound closure tests two months after showing high-blood-sugar syndromes.

### Trauma surgeries for the diabetic animals

i. The high-blood-sugar animals lower than 300g in weight were eliminated and the rest were randomized into groups.
ii. The animals were anesthetized with pentobarbital and the hairs on the surgical area (dorsal area) were removed. The surgical areas and instruments were then cleaned with alcohol before operation.
iii. The skins on the dorsal medium areas (4, 6 and 8 cm from the midpoint of two scapula) were excised (full thickness) using round cutting blades.

Measuring the area of wounds, applying medicaments, and preventing the wounds from receiving animal scratches
i. A standard ruler was placed beside the wounds and pictures were taken.
ii. The wounds were given testing medicaments.
iii. The wounds were covered with gauze and hoods were worn on the rats' necks.

Medicaments were applied to the rats twice a day (in the morning and evening) and the wounds were measured at each time point.

After the experiments were finished, the regenerated skins were taken for biochemistry and histology analysis.

### [Wound area analysis]

When taking pictures, a standard ruler was placed beside the wounds. Before analyzing the wounds with image pro, length was standardized with the standard ruler in the pictures to avoid the errors caused by different picturing distances.

### [Data analysis and statistics]

The areas of the three wounds on the rats' backs were analyzed by image pro. The original wound areas were the areas of day zero. The original wound areas were substrated by the wound areas at different time points and then divided by the original wound areas to get the wound closure percentages. The mean of the three wound closure percentages of each rat represents the wound closure of each rat. 4 to 7 rats per group were used for each test. The data was shown as mean ± standard error (SEM). The p-values of the testing results were calculated by t-test in statistics software sigma statis. P<0.05 means there is a significant difference, and it is marked with * on the statistics charts or tables. P<0.01 means there is a very significant difference, and it is marked with ** on the statistics charts or tables. P<0.001 means there is an extremely significant difference, and it is marked with
* * * on the statistics charts or tables.

### Example 6 Effects of Centella asiatica Urban extracts (S1) and Centella asiatica Urban extracts + Plectranthus amboinicus Benth crude extracts (PA) on wound closures in STZ-induced diabetic rats

*Plectranthus amboinicus* Benth crude extracts and ointments were prepared according to the methods described in Examples 1 and 4 respectively. The wound closure animal experiments were conducted according to Example 5. The results are shown in Figure 3 and Table 1.

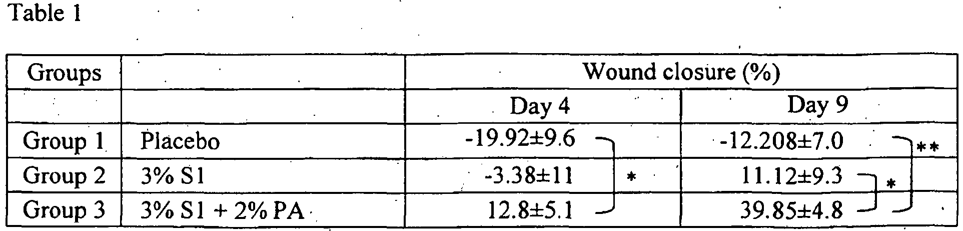

The results show that the STZ induced rats treated with *Plectranthus amboinicus* Benth crude extracts and *Centella* asiatica Urban extracts have better wound closure effects than those treated with *Centella asiatica* Urban extracts alone.

### Example 7 Effects of Centella asiatica Urban extracts (S1) and Centella asiatica Urban extracts + Plectranthus amboinicus Benth extracts extracted with different solvents (PA1, PA2 and PA3) on wound closures in STZ induced diabetic rats

Plectranthus *amboinicus* Benth extracts extracted with different solvents (PA1, PA2 and PA3) and ointments were prepared according to the methods described in Examples 2 and 4 respectively. The wound closure animal experiments were conducted according to Example 5. The results are shown in Figure 4. Upon comparison of the five experiment groups, it is found that the wound closure percentages of group 3 and group 5 on day 4 and 9 are higher than those of group 1 and group 2. Group 2 is significantly better than group 1 (P<0.05). The effect of group 4 is not significant. In summary, the groups ranked by wound closure effects in STZ induced diabetic rats from high to low are groups 5 and 3, group 2, and group 1.

PA1, PA2 and PA3 were extracted from dry *Plectranthus amboinicus* Benth of the same weight. Due to the different yield (as shown in Example 2), PA3 has the lowest yield and its dosage is the lowest in this test. However, the ointment made of PA3 and *Centella asiatica* Urban extracts has the best effect on wound closure. *Centella asiatica* Urban extracts + *Plectranthus amboinicus* Benth extracts extracted with different solvents have a positive effect on wound closure.

### Example 8 Effects of Centella asiatica Urban extracts (S1) and Centella asiatica Urban extracts + Plectranthus amboinicus Benth extracts (PAet and PA-F3) on wound closures in STZ-induced diabetic rats

*Plectranthus amboinicus* Benth extracts were prepared from alcohol extraction (PAet) and partial purification by column separation (PA-F3) according to methods described in Example 3. Ointments containing *Centella asiatica* Urban extracts + PAet and *Centella asiatica* Urban extracts + PA-F3 were prepared according to the methods described in Example 4. The wound closure animal experiments were conducted according to Example 5. The results are shown in Figure 5. Upon comparison of group 1 (placebo), group 2 (3% *Centella asiatica* Urban extracts), group 3 (3% *Centella asiatica* Urban extracts + PAet) and group 4 (3% *Centella asiatica* Urban extracts + PA-F3), it is found that although the means show that group 1 is better than group 2 on day 4, 9 and 14, there is no significant difference. On day 4 and 9, group 3 is significantly better than group 4. In summary, the groups ranked by wound closure effects in STZ-induced diabetic rats from high to low are group 4, groups 3 and 2, and group 1.

### Example 9. Effects of Centella asiatica Urban extracts (S1) and Centella asiatica Urban extracts + Plectranthus amboinicus Benth extracts (PA-F1, PA-F2, PA-F3 and PA-F4) on wound closures in STZ-induced diabetic rats

*Plectranthus amboinicus* Benth extracts (PA-F1, PA-F2, PA-F3 and PA-F4) were prepared from alcohol extraction and partial purification by column separation according to methods described in Example 3. Ointments containing *Plectranthus amboinicus* Benth extracts and *Centella asiatica* Urban extracts were prepared according to the methods described in Example 4. The wound closure animal experiments were conducted according to Example 5. The results are shown in Figure 6 and Table 2.

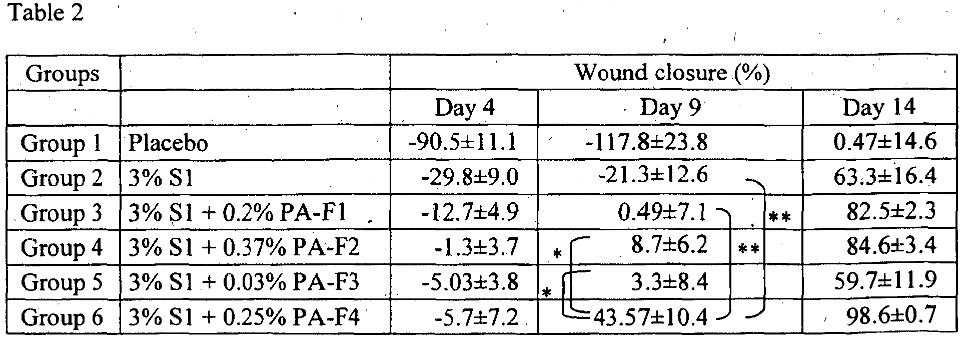

On day 9, wound closure percentage of group 6 is significantly higher than groups 2, 3, 4 and 5. In summary, the groups ranked by wound closure effects in STZ-induced diabetic rats from high to low are group 6, group 4, groups 5 and 3, group 2, and group 1. Especially, PA-F4 + *Centella asiatica* Urban extracts have a very significant effect on wound closure in diabetic rats.

### Example 10. Effects of 3% Centella asiatica Urban extracts (S1) and Plectranthus amboinicus Benth extracts of different concentration on wound closures in STZ-induced diabetic rats

Ointments containing 3% *Centella asiatica* Urban extracts and *Plectranthus* amboinicus Benth extracts of different concentration were prepared according to methods described in Examples 3 and 4. The wound closure animal experiments were conducted according to Example 5. The results are shown in Figure 7 and Table 3.

**Table 3**

| Groups | | Wound closure (%) | | |
|---|---|---|---|---|
| | | Day 4 | Day 9 | Day 14 |
| Group 1 | Placebo | -111.7±10.3 | -100.3±22 | 42.15±8.6 |
| Group 2 | 3% S1 | -42.04±4.4 | -50.23±11.3 | 74.6±5.3 |
| Group 3 | 0.18% PA-F4 | -18.80±8.4 | -19.53±14.6 | 83.78±4.7 |
| Group 4 | 3% S1 + 0.05% PA-F4 | -6.08±3.5 | -23.68±5.3 | 96.12±1.25 |
| Group 5 | 3% S1 + 0.18% PA-F4 | -8.5±3.1 | 41.79±6.3 | 95.1±3.0 |
| Group 6 | 3% S1 + 0.5% PA-F4 | -6.5±6.4 | 29.08±8.5 | 89.5±1.8 |

According to the data in Table 3, it is found that the wound closure percentages of groups 3, 4, 5 and 6 have significant to extremely significant differences compared to those of group 2 on day 4. On day 9, the wound closure percentages of groups 3, 4, 5 and 6 still have a significant difference compared to those of group 2. On day 14, only groups 4, 5 and 6 show significant to very significant differences in their wound closure percentages compared to group 2. Upon comparison of group 2 and group 3, it is found that the effect of group 3 is better than group 2 (having a significant difference on day 4); the combination effects are better. The results show that the effects on wound closures in diabetic rats of PA-F4 alone is better than that of *Centella asiatica* Urban extracts, and the combination of PA-F4 and *Centella asiatica* Urban extracts is much better than if they are used alone. The effective concentration of PA-F4 is 0.05% to 0.5 %, and the preferred concentration is 0.18%.

### Example 11 Effects of 0.26% PA-F4 extracts and Centella asiatica Urban extracts (S1) of different concentration on wound closures in STZ-induced diabetic rats

Ointments containing 0.26% PA-F4 and *Centella asiatica* Urban extracts of different concentration were prepared according to methods described in Examples 3 and 4. The wound closure animal experiments were conducted according to Example 5. The results are shown in Figure 8 and Table 4. On day 4, the wound closure percentages of groups 4, 5 and 6 have extremely significant differences compared to those of group 1. On day 9, the wound closure percentages of groups 4, 5 and 6 have significant or very significant differences compared to those of group 1. On day 14, the wound closure percentages of groups 4 and 5 have significant or very significant differences compared to those of group 1. The results show that the effects on wound closures in diabetic rats using the combination of 0.26% PA-F4 and 1 to 9% of *Centella asiatica* Urban extracts is better than using *Centella asiatica* Urban extracts alone. The preferred concentration of *Centella asiatica* Urban extracts is 1 to 3%.

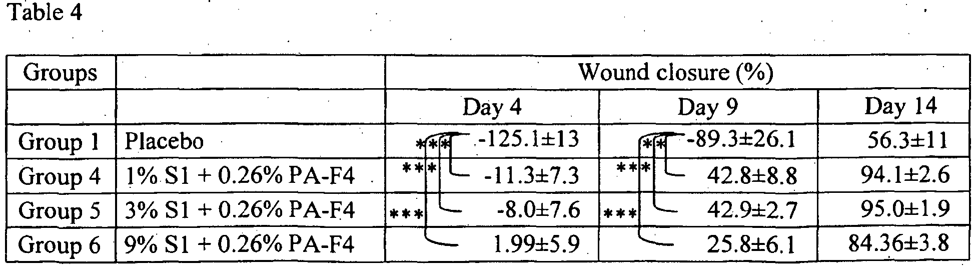

### Example 12 Effects of PA-F4 and 3% Centella asiatica Urban extracts (S1) on wound closures in db/db mice

Ointments containing 0.26% PA-F4 and *Centella asiatica* Urban extracts of different concentration were prepared according to methods described in Examples 3 and 4. The wound closure animal experiments were conducted according to Example 5. The results are shown in Figure 9 and Table 5. Upon comparison of the three experiments, the degree of wound closure in group 3 (3% *Centella asiatica* Urban extracts + 0.26% PA-F4) is significantly higher than group 2 (3% *Centella asiatica* Urban extracts), and very significantly higher than group 1 (placebo). The results show that 3% *Centella asiatica* Urban extracts + 0.26% PA-F4 has a better effect on wound closure in diabetic db/db mice than 3% *Centella asiatica* Urban extracts alone, and is much better than a placebo without any medicament.

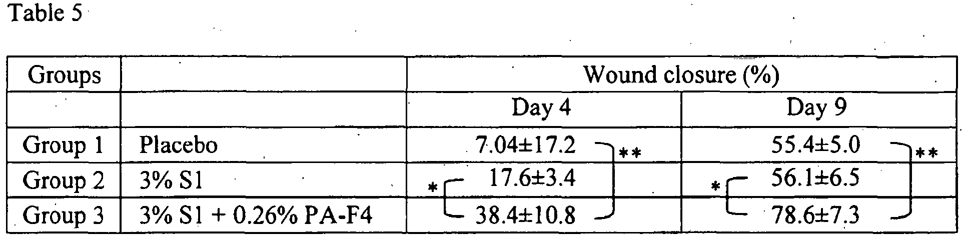

## Claims

1. A pharmaceutical composition for treating skin disorders comprising a therapeutically effective amount of *Plectranthus amboinicus* Benth crude extracts/extracts and a therapeutically effective amount of *Centella asiatica* Urban whole plant extracts comprising asiaticoside.

2. The pharmaceutical composition according to Claim 1, comprising a therapeutically effective amount of *Plectranthus amboinicus* Benth extracts

3. The pharmaceutical composition according to Claim 1 or 2, comprising at least one pharmaceutically acceptable carrier, diluent or excipient.

4. The pharmaceutical composition according to any one of the preceding claims wherein the weight ratio between *Plectranthus amboinicus* Benth crude extracts/extracts and *Centella asiatica* Urban extracts is about 1:60 to 1:4.

5. The pharmaceutical composition according to any one of the preceding claims, formulated as an ointment, solution or spray.

6. The pharmaceutical composition according to Claim 5, formulated as an ointment wherein the *Plectranthus amboinicus* Benth crude extracts/extracts are about at least 0.01% by weight of the ointment and the *Centella asiatica* Urban extracts are about at least 0.1% by weight of the ointment.

7. The pharmaceutical composition according to Claim 6, wherein the *Plectranthus amboinicus* Benth crude extracts/extracts are about 0.01% to 5% by weight of the ointment and the *Centella asiatica* Urban extracts are about 0.1% to 20% by weight of the ointment.

8. The pharmaceutical composition according to Claim 2, wherein the *Plectranthus amboinicus* Benth extracts are obtained from alcohol-dipped *Plectranthus amboinicus* Benth using column separation.

9. The pharmaceutical composition according to Claim 8, wherein the *Plectranthus amboinicus* Benth extracts have the following HPLC peak of retention time:
| Peak Retention time (min) | |
|---|---|
| 1 | 18.7 |
| 2 | 19.8 |
| 3 | 20.7 |
| 4 | 23.9 |
| 5 | 24.2 |
| 6 | 28.3 |
| 7 | 33.2 |
| 8 | 33.7 |
| 9 | 37.5 |
| 10 | 41.7 |
| 11 | 43.4 |
| 12 | 45.8 |
wherein said HPLC is conducted at the condition of:
Flow rate: 1.0ml/min Pressure Limit: 250 kgf/cm²
Sample amount: 10 µl
PDA condition: Sampling period: 0.64 sec
Wavelength range: 190-370 nm
Channel: 204, 254 nm
Elution Profile:
| Time (min) | | | | |
|---|---|---|---|---|
| Mobile phase | 0 | 10 | 55 | 60 |
| H2O | 90% | 90% | 20% | 80% |
| Acetonitrile | 10% | 10% | 80% | 20% |

10. The pharmaceutical composition according to Claim 8, wherein said extracts are separated by column chromatography comprising:
- washing with a high-polarity solvent and collecting the elution buffer;
- washing with a sub-high-polarity solvent having a five- to ten-fold volume of the dry material volume and collecting the elution buffer;
- washing with a medium-polarity solvent having a five- to ten-fold volume of the dry material volume and collecting the elution buffer; and
- washing with a low-polarity solvent having a five- to ten-fold volume of the dry material volume and collecting the elution buffer.

11. The pharmaceutical composition according to Claim 10, wherein the *Plectranthus amboinicus* Benth extracts collected by wash with low polar solvents having a five- to ten-fold volume of the dry material volume have the following HPLC peak of retention time:
| Peak Retention time (min) | |
|---|---|
| 1 | 11.1 |
| 2 | 11.4 |
| 3 | 16.9 |
| 4 | 17.3 |
| 5 | 22.9 |
| 6 | 24.1 |
| 7 | 25.1 |
| 8 | 26.6 |
| 9 | 27.7 |
| 10 | 28.5 |
| 11 | 29.3 |
| 12 | 29.6 |
| 13 | 30.4 |
| 14 | 31.3 |
| 15 | 33.0 |
wherein said HPLC is conducted at the condition of:
Flow rate: 1.0ml/min Pressure Limit: 250kgf/cm²
Sample amount: 10 µl
PDA conditions: Sampling period: 0.64 sec
Wavelength range: 190-370 nm
Channels: 270, 320 nm
Elution Profile:
| | Time (min) | | | |
|---|---|---|---|---|
| Mobile phase | 0 | 15 | 45 | 50 |
| n-hexane | 95% | 85% | 30% | 95% |
| Ethyl acetate | 5% | 15% | 70% | 5% |

12. The pharmaceutical composition according any one of the preceding claims for use in the treatment of general trauma and bedsores.

13. The pharmaceutical composition according to any one of the preceding claims for use in enhancing the healing of wounds for diabetic patients.

14. A wound dressing comprising a pharmaceutical composition according to any one of Claims 1 to 11.

15. The dressing according to Claim 14, formulated as a bandage with adhesive or patch.

16. A method for preparing the pharmaceutical composition of Claim 1, comprising the steps of:
- mixing *Plectranthus amboinicus* Benth crude extracts and *Centella asiatica* Urban whole plant extracts comprising asiaticoside, and
- optionally adding a pharmaceutically acceptable carrier, diluent or excipient,
wherein the *Plectranthus amboinicus* Benth crude extracts were prepared by
(a) extracting juices from whole *Plectranthus amboinicus* Benth plants directly; and
(b) drying the *Plectranthus amboinicus* Benth juices obtained in (a) to obtain the dry powders of *Plectranthus amboinicus* Benth crude extracts.

17. A method for preparing the pharmaceutical composition of Claim 2, comprising the steps of:
- mixing *Plectranthus amboinicus* Benth extracts and *Centella asiatica* Urban whole plant extracts comprising asiaticoside, and
- optionally adding a pharmaceutically acceptable carrier, diluent or excipient,
wherein the *Plectranthus amboinicus* Benth extracts were prepared by
(a) dipping *Plectranthus amboinicus* Benth dry materials in high-polarity solvent;
(b) concentrating the *Plectranthus amboinicus* Benth extract solution obtained in (a); and
(c) partially separating the concentrated *Plectranthus amboinicus* Benth extract solution obtained in (b) by three or four solvents with different polarity.

18. The method according to Claim 17 wherein the partial separation is conducted by column separation, which comprises:
- washing with a high-polarity solvent and collecting the elution buffer;
- washing with a sub-high-polarity solvent having a five- to ten-fold volume of the dry material volume and collecting the elution buffer;
- washing with a medium-polarity solvent having a five- to ten-fold volume of the dry material volume and collecting the elution buffer; or
- washing with a low-polarity solvent having a five- to ten-fold volume of the dry material volume and collecting the elution buffer.

19. Use of a combination of *Plectranthus amboinicus* Benth crude extracts/extracts and *Centella asiatica* Urban whole plant extracts comprising asiaticoside for the manufacture of a medicament for treating a skin disorder.

20. The use according to Claim 19, wherein the weight ratio between *Plectranthus amboinicus* Benth crude extracts/extracts and *Centella asiatica* Urban extracts is about 1:60 to 1:4.

21. The use according to Claim 19, wherein said medicament is formulated as an ointment, solution or spray.

22. The use according to Claim 19, wherein the medicament is formulated as an ointment and wherein the *Plectranthus amboinicus* Benth crude extracts/extracts are about at least 0.01% by weight of the ointment and the *Centella asiatica* Urban extracts are about at least 0.1 % by weight of the ointment.

23. The use according to Claim 22, wherein the *Plectranthus amboinicus* Benth crude extracts/extracts are about 0.01% to 5% by weight of the ointment and the *Centella asiatica* Urban extracts are about 0.1 % to 20% by weight of the ointment.

## Patentansprüche

1. Pharmazeutische Zusammensetzung zur Behandlung von Hautstörungen, welche eine therapeutisch wirksame Menge an *Plectranthus amboinicus* Benth Roh-Extrakt/-Extrakten und eine therapeutisch wirksame Menge an *Centella asiatica* Urban Gesamtpflanzen-Extrakten, welche Asiatikosid enthalten, umfasst.

2. Pharmazeutische Zusammensetzung nach Anspruch 1, welche eine therapeutisch wirksame Menge von *Plectranthus amboinicus* Benth Extrakten umfasst.

3. Pharmazeutische Zusammensetzung nach Anspruch 1 oder 2, welche mindestens ein(en) pharmazeutisch annehmbares(n) Träger, Verdünnungsmittel oder Exzipienten umfasst.

4. Pharmazeutische Zusammensetzung nach einem der vorstehenden Ansprüche, worin das Gewichtsverhältnis zwischen *Plectranthus amboinicus* Benth Roh-Extrakt-/Extrakten und *Centella asiatica* Urban Extrakten etwa 1:60 bis 1:4 beträgt.

5. Pharmazeutische Zusammensetzung nach einem der vorstehenden Ansprüche, formuliert als Salbe, Lösung oder Spray.

6. Pharmazeutische Zusammensetzung nach Anspruch 5, formuliert als Salbe, worin der/die *Plectranthus amboinicus* Benth Roh-Extrakt/-Extrakte mindestens etwa 0,01 Gew.-% der Salbe betragen und die *Centella asiatica* Urban Extrakte mindestens etwa 0,1 Gew.-% der Salbe betragen.

7. Pharmazeutische Zusammensetzung nach Anspruch 6, worin der/die *Plectranthus amboinicus* Benth Roh-Extrakt/-Extrakte etwa 0,01 bis 5 Gew.-% der Salbe betragen und die *Centella asiatica* Urban Extrakte etwa 0,1 bis 20 Gew.-% der Salbe betragen.

8. Pharmazeutische Zusammensetzung nach Anspruch 2, worin die *Plectranthus amboinicus* Benth Extrakte aus in Alkohol getauchten *Plectranthus amboinicus* Benth unter Verwendung einer Säulentrennung erhalten werden.

9. Pharmazeutische Zusammensetzung nach Anspruch 8, worin die *Plectranthus amboinicus* Benth Extrakte die folgenden HPLC Retentionszeit-Peaks aufweisen:
| | |
|---|---|
| 1 | 18.7 |
| 2 | 19.8 |
| 3 | 20.7 |
| 4 | 23.9 |
| 5 | 24.2 |
| 6 | 28.3 |
| 7 | 33.2 |
| 8 | 33.7 |
| 9 | 37.5 |
| 10 | 41.7 |
| 11 | 43.4 |
| 12 | 45.8 |
worin die HPLC unter folgenden Bedingungen durchgeführt wurde:
Strömungsrate : 1,0 ml /min, Druckgrenze: 250 kgf/cm²
Probenmenge: 10 µl
PDA-Bedingung : Probengewinnungs-Zeitspanne : 0,64 Sek.
Wellenlängenbereich: 190 - 370 nm
Kanäle: 204, 254 nm
Elutionsprofil:
| | Zeit (min) | | | |
|---|---|---|---|---|
| Mobile Phase | 0 | 10 | 55 | 60 |
| H₂O | 90% | 90% | 20% | 80% |
| Acetonitril | 10% | 10% | 80% | 20% |

10. Pharmazeutische Zusammensetzung nach Anspruch 8, worin die Extrakte mittels Säulenchromatographie getrennt werden, welche umfasst:
- Waschen mit einem hochpolarem Lösungsmittel und Gewinnen des Elutionspuffers;
- Waschen mit einem Lösungsmittel mit etwas geringerer Polarität mit einem fünf- bis zehn-fachen Volumen des Trockenmaterial-Volumens und Gewinnen des Elutionspuffers;
- Waschen mit einem Lösungsmittel mittlerer Polarität mit einem fünf- bis zehn-fachen Volumen des Trockenmaterial-Volumens und Gewinnen des Elutionspuffers; und
- Waschen mit einem Lösungsmittel geringer Polarität mit einem fünf- bis zehn-fachen Volumen des Trockenmaterial-Volumens und Gewinnen des Elutionspuffers.

11. Pharmazeutische Zusammensetzung nach Anspruch 10, worin die *Plectranthus amboinicus* Benth Extrakte, die durch Waschen mit Lösungsmitteln geringer Polarität mit einem fünf- bis zehn-fachen Volumen des Trockenmaterial-Volumens gewonnen wurden, die folgenden HPLC-Retentionszeit-Peaks aufweisen:
| | |
|---|---|
| 1 | 11.1 |
| 2 | 11.4 |
| 3 | 16.9 |
| 4 | 17.3 |
| 5 | 22.9 |
| 6 | 24.1 |
| 7 | 25.1 |
| 8 | 26.6 |
| 9 | 27.7 |
| 10 | 28.5 |
| 11 | 29.3 |
| 12 | 29.6 |
| 13 | 30.4 |
| 14 | 31.3 |
| 15 | 33.0 |
worin die HPLC unter folgenden Bedingungen durchgeführt wurde:
Strömungsrate : 1,0 ml /min, Druckgrenze: 250 kgf/cm²
Probenmenge: 10 µl
PDA-Bedingung : Probengewinnungs-Zeitspanne : 0,64 Sek.
Wellenlängenbereich: 190 - 370 nm
Kanäle: 270, 320 nm
Elutionsprofil:
| | Zeit (min) | | | |
|---|---|---|---|---|
| Mobile Phase | 0 | 15 | 45 | 50 |
| H₂O | 95% | 85% | 30% | 95% |
| Acetonitril | 5% | 15% | 70% | 5%. |

12. Pharmazeutische Zusammensetzung nach einem der vorstehenden Ansprüche zur Verwendung bei der Behandlung von allgemeinem Trauma und Wundliegen.

13. Pharmazeutische Zusammensetzung nach einem der vorstehenden Ansprüche zur Steigerung der Wundheilung bei Diabetes-Patienten.

14. Wundverband, welcher eine pharmazeutische Zusammensetzung nach einem der Ansprüche 1 bis 11 umfasst.

15. Wundverband nach Anspruch 14, formuliert als Verband mit Klebstoff oder als Pflaster.

16. Verfahren zur Herstellung einer pharmazeutischen Zusammensetzung nach Anspruch 1, welche die Schritte umfasst:
- Mischen von *Plectranthus amboinicus* Benth Roh-Extrakten und *Centella asiatica* Urban Gesamtpflanzen-Extrakten, welche Asiatikosid enthalten, und
- wahlweise Zusetzen eines pharmazeutisch annehmbaren Trägers, Verdünnungsmittels oder Exzipienten,
worin die *Plectranthus amboinicus* Benth Roh-Extrakte hergestellt wurden durch
(a) direktes Extrahieren von Säften aus gesamten *Plectranthus amboinicus* Benth Pflanzen; und
(b) Trocknen der in Schritt (a) erhaltenen *Plectranthus amboinicus* Benth Säfte, um das Trockenpulver der *Plectranthus amboinicus* Benth Extrakte zu erhalten.

17. Verfahren zur Herstellung der pharmazeutischen Zusammensetzung nach Anspruch 2, welches die Schritte umfasst:
- Mischen von *Plectranthus amboinicus* Benth Extrakten und *Centella asiatica* Urban Gesamtpflanzen-Extrakten, welche Asiatikosid enthalten, und
- Wahlweise Zusetzen eines pharmazeutisch annehmbaren Trägers, Verdünnungsmittels oder Exzipienten,
worin die *Plectranthus amboinicus* Benth Extrakte hergestellt wurden durch
(a) Tauchen von *Plectranthus amboinicus* Benth Trockenmaterialien in ein hochpolares Lösungsmittel;
(b) Konzentrieren der in Schritt (a) erhaltenen *Plectranthus amboinicus* Benth Extrakt-Lösung; und
(c) Teilweises Auftrennen der in Schritt (b) erhaltenen *Plectranthus amboinicus* Benth Extrakt-Lösung mit drei oder vier Lösungsmitteln unterschiedlicher Polarität.

18. Verfahren nach Anspruch 17, wobei die teilweise Trennung durchgeführt wird durch Säulentrennung, welche umfasst:
- Waschen mit einem hochpolarem Lösungsmittel und Gewinnen des Elutionspuffers;
- Waschen mit einem Lösungsmittel mit etwas geringerer Polarität mit einem fünf- bis zehn-fachen Volumen des Trockenmaterial-Volumens und Gewinnen des Elutionspuffers;
- Waschen mit einem Lösungsmittel mit mittlerer Polarität mit einem fünf- bis zehn-fachen Volumen des Trockenmaterial-Volumens und Gewinnen des Elutionspuffers; oder Waschen mit einem Lösungsmittel mit geringer Polarität mit einem fünf- bis zehn-fachen Volumen des Trockenmaterial-Volumens und Gewinnen des Elutionspuffers.

19. Verwendung einer Kombination von *Plectranthus amboinicus* Benth Roh-Extrakt/- Extrakten und *Centella asiatica* Urban Gesamtpflanzen-Extrakten, welche Asiatikosid enthalten, zur Herstellung eines Medikaments zur Behandlung von Hautstörungen.

20. Verwendung nach Anspruch 19, wobei das das Gewichtsverhältnis zwischen *Plectranthus amboinicus* Benth Roh-Extrakt/-Extrakten und *Centella asiatica* Urban Extrakten 1:60 bis 1:4 beträgt.

21. Verwendung nach Anspruch 19, wobei das Medikament als Salbe, Lösung oder Spray formuliert ist.

22. Verwendung nach Anspruch 19, wobei das Medikament als Salbe formuliert ist, und worin der/die *Plectranthus amboinicus* Benth Roh-Extrakt/-Extrakte mindestens etwa 0,01 Gew.-% der Salbe betragen und die *Centella asiatica* Urban Extrakte mindestens etwa 0,1 Gew.-% der Salbe betragen.

23. Verwendung nach Anspruch 22, wobei der/die *Plectranthus amboinicus* Benth RohExtrakt-/Extrakten etwa 0,01 bis 5 Gew.-% der Salbe betragen und die *Centella asiatica* Urban Extrakte etwa 0,1 bis 20 Gew.-% der Salbe betragen.

## Revendications

1. Composition pharmaceutique destinée au traitement de troubles cutanés comprenant une quantité efficace sur le plan thérapeutique d'extraits bruts/extraits de Plectranthus amboinicus Benth et une quantité efficace sur le plan thérapeutique d'extraits de plante entière de Centella asiatica Urbaine comprenant de l'asiaticoside.

2. Composition pharmaceutique selon la revendication 1, comprenant une quantité efficace sur le plan thérapeutique d'extraits de Plectranthus amboinicus Benth.

3. Composition pharmaceutique selon la revendication 1 ou 2, comprenant au moins un véhicule, diluant ou excipient acceptable sur le plan pharmaceutique.

4. Composition pharmaceutique selon l'une quelconque des revendications précédentes, dans laquelle le rapport pondéral entre les extraits bruts/extraits de Plectranthus amboinicus Benth et les extraits de Centella asiatica Urbaine va d'environ 1:60 à 1:4.

5. Composition pharmaceutique selon l'une quelconque des revendications précédentes, formulée en tant que pommade, solution ou aérosol.

6. Composition pharmaceutique selon la revendication 5, formulée en tant que pommade, dans laquelle les extraits bruts/extraits de Plectranthus amboinicus Benth représentent environ au moins 0,01 % en poids de la pommade et les extraits de Centella asiatica Urbaine représentent environ au moins 0,1 % en poids de la pommade.

7. Composition pharmaceutique selon la revendication 6, dans laquelle les extraits bruts/extraits de Plectranthus amboinicus Benth représentent environ 0,01 % à 5 % en poids de la pommade et les extraits de Centella asiatica Urbaine représentent environ 0,1 % à 20 % en poids de la pommade.

8. Composition pharmaceutique selon la revendication 2, dans laquelle les extraits de Plectranthus amboinicus Benth sont obtenus à partir de Plectranthus amboinicus Benth trempé dans de l'alcool au moyen d'une séparation sur colonne.

9. Composition pharmaceutique selon la revendication 8, dans laquelle les extraits de Plectranthus amboinicus Benth présentent le pic HPLC suivant de temps de rétention :
Temps de rétention de pic (min)
| | |
|---|---|
| 1 | 18,7 |
| 2 | 19,8 |
| 3 | 20,7 |
| 4 | 23,9 |
| 5 | 24,2 |
| 6 | 28,3 |
| 7 | 33,2 |
| 8 | 33,7 |
| 9 | 37,5 |
| 10 | 41,7 |
| 11 | 43,4 |
| 12 | 45,8 |
dans laquelle ladite HPLC est effectuée dans les conditions suivantes :
Débit : 1,0 mL/min
Limite de pression : 250 kgf/cm2
Quantité d'échantillon : 10 µL
Condition PDA : Période d'échantillonnage : 0,64 s
Intervalle de longueur d'onde : 190 à 370 nm
Canal : 204, 254 nm
Profil d'élution :
Temps (min)
| | | | | |
|---|---|---|---|---|
| Phase mobile | 0 | 10 | 55 | 60 |
H2O 90%90%20%80%
Acétonitrile 10 % 10 % 80 % 20 %

10. Composition pharmaceutique selon la revendication 8, dans laquelle lesdits extraits sont séparés par une chromatographie sur colonne comprenant :
- le lavage avec un solvant à haute polarité et le recueil du tampon d'élution ;
- le lavage avec un solvant de polarité moyennement élevée possédant un volume de cinq à dix fois le volume de matériau sec et le recueil du tampon d'élution ;
- le lavage avec un solvant de polarité moyenne possédant un volume de cinq à dix fois le volume de matériau sec et le recueil du tampon d'élution ; et
- le lavage avec un solvant de basse polarité possédant un volume de cinq à dix fois le volume de matériau sec et le recueil du tampon d'élution ;

11. Composition pharmaceutique selon la revendication 10, dans laquelle les extraits de Plectranthus amboinicus Benth recueillis par lavage avec des solvants à basse polarité possédant un volume de cinq à dix fois le volume du matériau sec présentent le pic HPLC suivant de temps de rétention : Temps de rétention de pic (min)
| | |
|---|---|
| 1 | 11,1 |
| 2 | 11,4 |
| 3 | 16,9 |
| 4 | 17,3 |
| 5 | 22,9 |
| 6 | 24,1 |
| 7 | 25,1 |
| 8 | 26,6 |
| 9 | 27,7 |
| 10 | 28,5 |
| 11 | 29,3 |
| 12 | 29,6 |
| 13 | 30,4 |
| 14 | 31,3 |
| 15 | 33,0 |
dans laquelle ladite HPLC est effectuée dans les conditions suivantes :
Débit : 1,0 mL/minLimite de pression : 250 kgf/cm2
Quantité d'échantillon : 10 µL
Conditions PDA : Période d'échantillonnage : 0,64 s
Intervalle de longueur d'onde : 190 à 370 nm
Canaux : 270, 320 nm
Profil d'élution :
Temps (min)
| | | | | |
|---|---|---|---|---|
| Phase mobile | 0 | 15 | 45 | 50 |
| n-hexane | 95 % | 85 % | 30 % | 95 % |
| Acétate d'éthyle | 5 % | 15 % | 70 % | 5 % |

12. Composition pharmaceutique selon l'une quelconque des revendications précédentes, utilisable pour le traitement de traumatismes généraux et d'escarres de décubitus.

13. Composition pharmaceutique selon l'une quelconque des revendications précédentes, utilisable pour améliorer la guérison de blessures pour des patients diabétiques.

14. Pansement pour plaie comprenant une composition pharmaceutique selon l'une quelconque des revendications 1 à 11.

15. Pansement selon la revendication 14, formulé en tant que bandage avec adhésif ou patch.

16. Procédé de préparation de la composition pharmaceutique selon la revendication 1, comprenant les étapes consistant à :
- mélanger des extraits bruts de Plectranthus amboinicus Benth et des extraits de plante entière de Centella asiatica Urbaine comprenant de l'asiaticoside, et
- ajouter éventuellement un véhicule, diluent ou excipient acceptable sur le plan pharmaceutique,
dans lequel les extraits bruts de Plectranthus amboinicus Benth ont été préparés par
(a) extraction directe de jus de plantes entières de Plectranthus amboinicus Benth ; et
(b) séchage des jus de Plectranthus amboinicus Benth obtenus en (a) de façon à obtenir les poudres sèches d'extraits bruts de Plectranthus amboinicus Benth.

17. Procédé de préparation de la composition pharmaceutique selon la revendication 2, comprenant les étapes consistant à :
- mélanger des extraits de Plectranthus amboinicus Benth et des extraits de plante entière de Centella asiatica Urbaine comprenant de l'asiaticoside, et
- ajouter éventuellement un véhicule, diluent ou excipient acceptable sur le plan pharmaceutique, dans lequel les extraits de Plectranthus amboinicus Benth ont été préparés par
(a) immersion de matériaux secs de Plectranthus amboinicus Benth dans un solvant à haute polarité ;
(b) concentration de la solution d'extrait de Plectranthus amboinicus Benth obtenue en (a) ; et
(c) séparation partielle de la solution d'extrait de Plectranthus amboinicus Benth concentrée obtenue en (b) par trois ou quatre solvants avec une polarité différente.

18. Procédé selon la revendication 17, dans lequel la séparation partielle est effectuée par une séparation sur colonne, qui comprend :
- le lavage avec un solvant à haute polarité et le recueil du tampon d'élution ;
- le lavage avec un solvant de polarité moyennement élevée possédant un volume de cinq à dix fois le volume de matériau sec et le recueil du tampon d'élution ;
- le lavage avec un solvant de polarité moyenne possédant un volume de cinq à dix fois le volume de matériau sec et le recueil du tampon d'élution ; ou
- le lavage avec un solvant de basse polarité possédant un volume de cinq à dix fois le volume de matériau sec et le recueil du tampon d'élution ;

19. Utilisation d'une combinaison d'extraits bruts/extraits de Plectranthus amboinicus Benth et d'extraits de plante entière de Centella asiatica Urbaine comprenant de l'asiaticoside pour la fabrication d'un médicament destiné au traitement d'un trouble cutané.

20. Utilisation selon la revendication 19, dans laquelle le rapport pondéral entre les extraits bruts/extraits de Plectranthus amboinicus Benth et les extraits de Centella asiatica Urbaine va d'environ 1:60 à 1:4.

21. Utilisation selon la revendication 19, dans laquelle ledit médicament est formulé en tant que pommade, solution ou aérosol.

22. Utilisation selon la revendication 19, dans laquelle le médicament est formulé en tant que pommade et dans laquelle les extraits bruts/extraits de Plectranthus amboinicus Benth représentent environ au moins 0,01 % en poids de la pommade et les extraits de Centella asiatica Urbaine représentent environ au moins 0,1 % en poids de la pommade.

23. Utilisation selon la revendication 22, dans laquelle les extraits bruts/extraits de Plectranthus amboinicus Benth représentent environ 0,01 % à 5 % en poids de la pommade et les extraits de Centella asiatica Urbaine représentent environ 0,1 % à 20 % en poids de la pommade.
